(19) Europäisches Patentamt
European Patent Office
Office européen des brevets

(11) Numéro de publication: **0 261 004 B1**

(12) # FASCICULE DE BREVET EUROPEEN

(45) Date de publication de fascicule du brevet: **26.05.93**

(51) Int. Cl.[5]: **C07D 487/04**, A61K 31/415, C07D 471/04, C07D 233/84, C07D 233/64, C07D 233/70, C07D 233/54

(21) Numéro de dépôt: **87401777.5**

(22) Date de dépôt: **30.07.87**

(54) **Nouveaux dérivés de l'imidazole et leurs sels, leur procédé de préparation, leur application comme médicaments et les compositions les renfermant.**

(30) Priorité: **18.08.86 GB 8620060**

(43) Date de publication de la demande:
**23.03.88 Bulletin 88/12**

(45) Mention de la délivrance du brevet:
**26.05.93 Bulletin 93/21**

(84) Etats contractants désignés:
**CH DE FR IT LI NL**

(56) Documents cités:
**EP-A- 0 061 380**
**EP-A- 0 062 580**
**EP-A- 0 147 312**
**EP-A- 0 217 142**
**GB-A- 2 128 989**

(73) Titulaire: **ROUSSEL-UCLAF**
**35, Boulevard des Invalides**
**F-75007 Paris(FR)**

(72) Inventeur: **Fenske, Dankwart Christopher**
**17 Waveley Road**
**Weybridge Surrey, KT13 8UT(GB)**
Inventeur: **Kuo, Elizabeth Anne**
**1 Sandpiper Bridge**
**Covingham Swindon Wiltshire(GB)**
Inventeur: **Tully, Wilfred Roger**
**Abbey Cottage 1 Grove Court The Waterloo**
**Cirencester GL7, 2PZ, Gloucestershire(GB)**

(74) Mandataire: **Vieillefosse, Jean-Claude et al**
**Département des Brevets ROUSSEL UCLAF**
**B.P no 9**
**F-93230 Romainville (FR)**

**EP 0 261 004 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés de l'imidazole et leurs sels, leur préparation, leur application à titre de médicaments et les compositions les renfermant.

L'invention a pour objet les nouveaux dérivés de l'imidazole et leurs sels d'addition avec les acides, caractérisés en ce qu'ils répondent à la formule (I) :

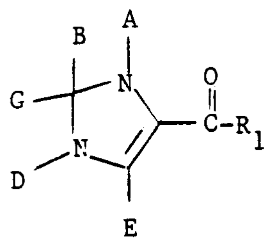

(I)

dans laquelle $R_1$ représente soit un radical

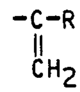

dans lequel R représente un atome d'hydrogène ou un radical alcoyle renfermant de 1 à 5 atomes de carbone, soit un radical $-C\equiv C-R'$ dans lequel $R'$ représente un atome d'hydrogène, un radical alcoyle renfermant de 1 à 5 atomes de carbone, un radical hydroxyalcoyle renfermant de 1 à 5 atomes de carbone ou un radical aryle renfermant de 6 à 10 atomes de carbone non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone, et

- soit A représente un radical alcoyle renfermant de 1 à 5 atomes de carbone et B et G ensemble avec l'atome de carbone auquel ils sont liés représentent un radical carbonyle ou thiocarbonyle et D représente un radical alcoyle renfermant de 1 à 5 atomes de carbone ou un radical phényle non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone,
- soit A et B représentent ensemble une liaison et G représente un radical alcoyle, alcoxy, alkylthio, alkylsulfinyle ou alkylsulfonyle renfermant de 1 à 5 atomes de carbone, D représente un radical alcoyle renfermant de 1 à 5 atomes de carbone ou un radical phényle non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone,
- soit A et B représentent ensemble une liaison et G et D ensemble représentent un groupe de formule :

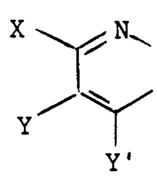

ou

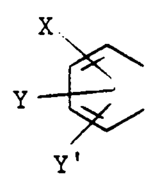

dans laquelle X représente un radical alcoxy ou alkylthio et Y et $Y'$, identiques ou différents, représentent un atome d'hydrogène ou un radical alcoyle renfermant de 1 à 5 atomes de carbone, E représente un atome d'hydrogène ou d'halogène, étant entendu que lorsque A et B ensemble représentent une liaison et G et D ensemble représentent un groupe

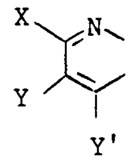

E ne peut pas représenter un atome d'hydrogène si $R_1$ représente un radical

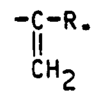

Dans les composés de formule (I), lorsque A et B ensemble représentent une liaison et G et D ensemble représentent un groupe

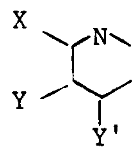

ou

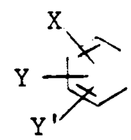

il est entendu que les composés de formule (I) résultants possèdent 2 structures possibles à savoir :

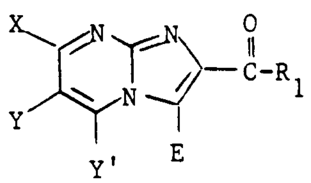

ou

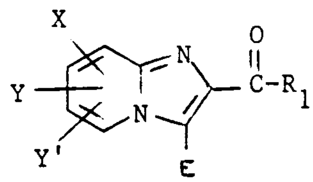

respectivement.

Dans la formule générale (I) et dans ce qui suit, le terme radical alcoyle renfermant de 1 à 5 atomes de carbone désigne, par exemple, un radical méthyle, éthyle, propyle, isopropyle, butyle linéaire ou ramifié ou pentyle linéaire ou ramifié ; le terme radical alcoxy renfermant de 1 à 5 atomes de carbone désigne, par exemple, un radical méthoxy, éthoxy, propoxy, isopropoxy, butoxy linéaire ou ramifié, pentyloxy linéaire ou ramifié ; le terme hydroxyalcoyle renfermant de 1 à 5 atomes de carbone désigne par exemple un radical hydroxyméthyle, hydroxyéthyle, hydroxy-n-propyle, hydroxyisopropyle, hydroxybutyle linéaire ou ramifié, hydroxypentyle linéaire ou ramifié.

Le terme aryle renfermant de 6 à 10 atomes de carbone désigne par exemple un radical phényle ou naphtyle, chacun d'entre eux pouvant être éventuellement substitué comme mentionné ci-dessus.

Le terme alkylthio renfermant de 1 à 5 atomes de carbone désigne, par exemple, un radical méthylthio, éthylthio, n-propylthio, isopropylthio, butylthio linéaire ou ramifié, pentylthio linéaire ou ramifié.

Le terme alkylsulfinyle renfermant de 1 à 5 atomes de carbone désigne, par exemple, un radical méthylsulfinyle, éthylsulfinyle, n-propylsulfinyle ou isopropylsulfinyle, butylsulfinyle linéaire ou ramifié, pentylsulfinyle linéaire ou ramifié.

Le terme alkylsulfonyle renfermant de 1 à 5 atomes de carbone désigne, par exemple, un radical méthylsulfonyle, éthylsulfonyle, propylsulfonyle, isopropylsulfonyle, butylsulfonyle linéaire ou ramifié, pentylsulfonyle linéaire ou ramifié.

Les sels d'addition avec les acides minéraux ou organiques peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, alcane sulfoniques, tels que l'acide méthanesulfonique ou arylsulfoniques, tels que l'acide benzènesulfonique.

Parmi les produits, objet de l'invention, on peut citer notamment les dérivés répondant à la formule (I) ci-dessus, ainsi que leurs sels, caractérisés en ce que dans ladite formule (I), $R_1$ représente un radical

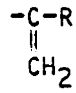

dans lequel R est défini comme ci – dessus ou un radical $-C \equiv C-R'$ dans lequel R′ est défini comme ci – dessus et E représente un atome d'halogène.

Parmi les produits, objet de l'invention, on retient plus particulièrement les dérivés répondant à la formule (I) ci – dessus ainsi que leurs sels, caractérisés en ce que dans ladite formule (I), A et B ensemble représentent une liaison et G et D ensemble représentent un groupe de formule :

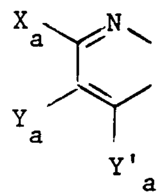

ou

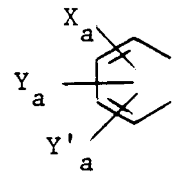

dans lequel $X_a$ représente un radical méthoxy ou méthylthio et $Y_a$ et $Y'_a$, identiques ou différents, représentent un atome d'hydrogène ou un radical méthyle ou éthyle, et E représente un atome d'halogène.

Parmi les produits, objet de l'invention, on retient également plus particulièrement les dérivés répondant à la formule (I) ci – dessus ainsi que leurs sels, caractérisés en ce que dans ladite formule (I), A et B ensemble représentent une liaison, G représente un radical alkylthio et D représente un radical phényle non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone.

Parmi les produits, objet de l'invention, on retient tout spécialement les dérivés répondant à la formule (I) ci – dessus dont les noms suivent :

- la 1 – (3 – bromo – 6 – éthyl – 5 – méthyl – 7 – méthylthioimidazo [1,2 – a] pyrimidin – 2 – yl) 2 – méthyl 2 – propèn – 1 – one ;
- la 1 – [5 – bromo – 1 – (4 – éthylphényl) – 2 – méthylthioimidazol – 4 – yl] 2 – méthyl 2 – propèn – 1 – one ;
- la 1 – [5 – bromo – 1 – (4 – méthoxyphényl) – 2 – méthylthioimidazol – 4 – yl] 2 – propèn – 1 – one ;
- la 1 – (3 – bromo – 6 – éthyl – 7 – méthoxy – 5 – méthylimidazo [1,2 – a] pyrimidin – 2 – yl) 2 – propèn – 1 – one ; et
- la 1 – (3 – bromo – 6 – éthyl – 7 – méthoxy – 5 – méthylimidazo [1,2 – a] pyrimidin – 2 – yl) 3 – phényl 2 – propyn – 1 – one ;

et leurs sels d'addition avec les acides.

L'invention a également pour objet un procédé de préparation des produits tels que définis par la formule générale (I), répondant à la formule :

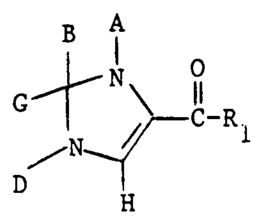

dans laquelle A, B, G, D et $R_1$ sont définis comme précédemment ainsi que de leurs sels, caractérisés en ce que l'on soumet un produit de formule (II) :

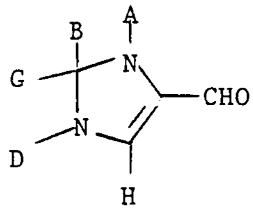

(II)

dans laquelle A, B, G et D sont définis comme précédemment à un générateur de carbanions $R_1^-$ tel qu'un réactif de Grignard de formule (III) :

$R_1 - Mg - Br$  (III)

ou un alkyllithien de formule ($III_A$) :

$R_1 - Li$  ($III_A$)

formules dans lesquelles $R_1$ est défini comme précédemment pour obtenir un produit de formule (IV) :

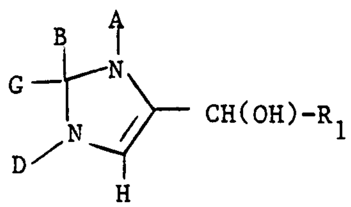

(IV)

dans laquelle A, B, G, D et $R_1$ sont définis comme précédemment que l'on soumet à une oxydation pour obtenir le produit de formule générale (I) dans laquelle E représente un atome d'hydrogène que, le cas échéant, l'on salifie.

Dans des conditions préférentielles de mise en oeuvre de l'invention, le procédé de préparation ci-dessus décrit est caractérisé en ce que :

- la réaction du produit de formule (II) avec le produit de formule (III) ou ($III_A$) est effectuée au sein d'un solvant organique tel que le tétrahydrofuranne ;
- l'oxydation du produit de formule (IV) est effectuée de manière conventionnelle, par exemple à l'aide de dioxyde de manganèse ;
- la réaction est effectuée au sein d'un solvant organique tel que le chloroforme à la température de reflux du milieu réactionnel.

L'invention a également pour objet un procédé de préparation des produits tels que définis par la formule générale (I) répondant à la formule ($I_B$) :

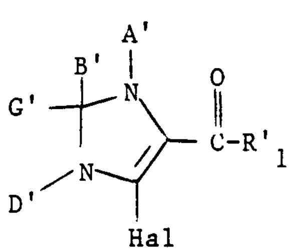

($I_B$)

dans laquelle $R'_1$ représente un radical

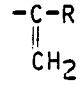

dans lequel R est défini comme précédemment ou un radical $-C \equiv C-R'$ dans lequel R′ est défini comme précédemment et

- soit A′ représente un radical alcoyle renfermant de 1 à 5 atomes de carbone et B′ et G′ ensemble avec l'atome de carbone auquel ils sont liés représentent un radical carbonyle ou thiocarbonyle et D′ représente un radical alcoyle renfermant de 1 à 5 atomes de carbone ou un radical phényle non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone,
- soit A′ et B′ représentent ensemble une liaison et G′ représente un radical alcoyle ou alkylthio, renfermant de 1 à 5 atomes de carbone, et D′ représente un radical alcoyle renfermant de 1 à 5 atomes de carbone ou un radical phényle non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone,
- soit A′ et B′ représentent ensemble une liaison et G′ et D′ ensemble représentent un groupe de formule :

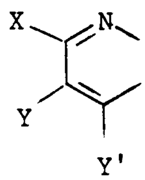

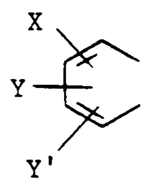

dans laquelle X, Y et Y′ sont définis comme précédemment et Hal représente un atome d'halogène, ainsi que de leurs sels, caractérisé en ce que l'on traite un produit de formule ($I_A$) :

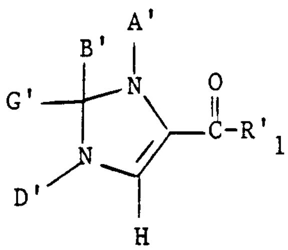

($I_A$)

dans laquelle A′, B′, G′, D′ et $R'_1$ sont définis comme ci-dessus par un N-halosuccinimide au sein d'un solvant organique tel que le chloroforme ou le tétrachlorure de carbone pour obtenir un produit de formule ($I_B$) que, le cas échéant, l'on salifie.

Certains produits de formule ($I_A$) utilisés au départ sont des produits de formule générale (I) et peuvent être préparés selon le procédé décrit précédemment.

L'invention a également pour objet un procédé de préparation des produits tels que définis par la formule (I) ci-dessus répondant à la formule ($I_D$) :

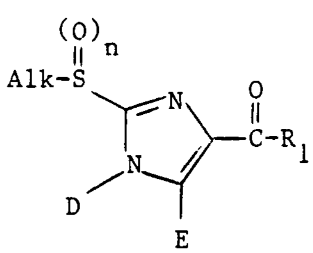

($I_D$)

dans laquelle $R_1$, D et E sont définis comme précédemment, Alk représente un radical alcoyle renfermant de 1 à 5 atomes de carbone et n a la valeur 1 ou 2 ainsi que de leurs sels, caractérisé en ce que l'on oxyde un produit de formule ($I_C$) :

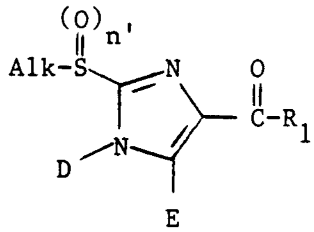

$(I_C)$

dans laquelle $R_1$, D, E et Alk sont définis comme ci-dessus et n′ a la valeur 0 ou 1 pour obtenir un produit de formule $(I_D)$ que, le cas échéant, l'on salifie.

Dans des conditions préférentielles de mise en oeuvre, le procédé de préparation ci-dessus décrit est caractérisé en ce que l'oxydation est effectuée de préférence à l'aide de métapériodate de sodium en présence d'un solvant organique tel qu'une solution aqueuse d'un alcanol de bas poids moléculaire comme le méthanol à la température de 50¤C par exemple.

Il faut noter que pour préparer des produits de formule $(I_D)$ dans laquelle n = 1 (c'est-à-dire G représente un radical alkylsulfinyle) par le procédé décrit ci-dessus, la réaction sera limitée nécessairement au traitement du produit correspondant de formule $(I_C)$ dans laquelle n′ = 0 par un équivalent d'agent d'oxydation.

Les produits de formule $(I_D)$ dans laquelle n = 2 (c'est-à-dire G représente un radical sulfonyle) peuvent être préparés en principe par le procédé décrit ci-dessus, selon 2 voies.

On peut soit traiter un produit correspondant de formule $(I_C)$ dans laquelle n′ = 0 par deux ou plusieurs équivalents d'agent d'oxydation, soit traiter un produit correspondant de formule $(I_C)$ dans laquelle n′ = 1 par un ou plusieurs équivalents d'agent d'oxydation.

L'invention a encore pour objet un procédé de préparation (procédé D) des produits tels que définis par la formule (I) ci-dessus dans laquelle l'un au moins des substituants R1 et D comprend un radical hydroxy, caractérisé en ce que l'on déprotège le produit correspondant de formule (V) :

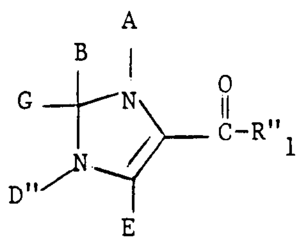

(V)

dans laquelle A, B, G et E sont définis comme précédemment, D" a soit la signification indiquée pour D, soit lorsque l'on veut obtenir un produit de formule (I) dans laquelle D représente un radical hydroxy libre, D" représente un précurseur de D contenant un radical hydroxy protégé et $R''_1$ a soit la signification indiquée pour $R_1$, soit lorsque l'on veut obtenir un produit de formule (I) dans laquelle $R_1$ représente un radical hydroxy libre, $R''_1$ représente un précurseur de $R_1$ contenant un radical hydroxy protégé, étant entendu que l'un au moins de D″ et de R″ représente respectivement un précurseur de D et de $R_1$ contenant un radical hydroxy protégé pour obtenir un produit de formule (I) dans laquelle $R_1$ et D sont définis comme ci-dessus que, le cas échéant, l'on salifie.

Les produits de formule (V) peuvent être préparés selon un des modes opératoires indiqués précédemment en utilisant les produits de départ appropriés dans lesquels les substituants $R_1$ et/ou D contiennent des radicaux hydroxy protégés.

Certains produits selon l'invention possèdent des radicaux hydroxy libres et il peut être nécessaire ou avantageux de les protéger lors de la préparation de ces produits.

Dans la plupart des cas, le groupe protecteur sera présent au cours de la synthèse et sera éliminé au stade final par des moyens appropriés comme l'hydrolyse par exemple.

Dans le cas où R′ représente un radical hydroxyalkyle renfermant de 1 à 5 atomes de carbone, le groupe protecteur est choisi parmi ceux couramment utilisés pour protéger les radicaux hydroxy aliphatiques comme par exemple le groupe tétrahydropyrannyle, mais d'autres groupes protecteurs connus de l'homme de l'art peuvent être utilisés.

Lorsque l'on fait réagir sur le produit de formule (II) un produit de formule (III) ou ($III_A$) dans laquelle le substituant $R_1$ comprend un groupe $R'$ représentant un radical hydroxyalkyle protégé, on obtient le produit protégé de formule (IV) correspondant, puis on poursuit la synthèse selon l'un des procédés décrits précédemment, en conservant la protection du radical hydroxy, protection que l'on élimine au cours du dernier stade. Un tel procédé est décrit dans la partie expérimentale.

Il en est de même, dans le cas où $R'$ représente un radical aryle renfermant de 6 à 10 atomes de carbone substitué par un radical hydroxy. Le groupement protecteur est choisi parmi ceux couramment utilisés pour ce type de groupement, par exemple un radical benzyle, mais d'autres groupes protecteurs connus de l'homme de l'art peuvent être également utilisés.

Les produits de formule (I) présentent un caractère basique.

On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques ; ils sont doués notamment de propriétés anti-inflammatoires, notamment par leur aptitude à modérer la fonction cellulaire inflammation/immunité par ralentissement de la libération d'acide arachidonique et par voie de conséquence la biosynthèse eicosanoïde.

Ces propriétés sont illustrées plus loin dans la partie expérimentale.

Ces propriétés justifient l'utilisation des nouveaux dérivés de l'imidazole répondant à la formule (I), ainsi que de leurs sels pharmaceutiquement acceptables, à titre de médicaments.

La présente invention a ainsi également pour objet l'application à titre de médicaments des nouveaux dérivés de l'imidazole répondant à la formule générale (I) ainsi que de leurs sels d'addition avec les acides pharmaceutiquement acceptables.

Parmi ces médicaments, objet de l'invention, on retient notamment les médicaments, caractérisés en ce qu'ils sont constitués par les nouveaux dérivés de l'imidazole répondant à la formule (I) dans laquelle $R_1$ représente un radical

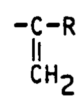

dans lequel R est défini comme ci-dessus ou un radical $-C{\equiv}C-R'$ dans lequel $R'$ est défini comme ci-dessus et E représente un atome d'halogène ainsi que leurs sels d'addition avec les acides pharmaceutiquement acceptables.

Parmi les médicaments de l'invention, on retient tout particulièrement ceux répondant à la formule (I), dans laquelle A et B ensemble représentent une liaison et G et D ensemble représentent un groupe de formule :

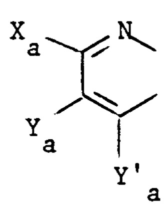

ou

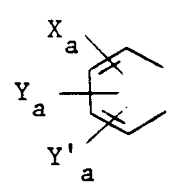

dans laquelle $X_a$ représente un radical méthoxy ou méthylthio et $Y_a$ et $Y'_a$, identiques ou différents, représentent un atome d'hydrogène ou un radical méthyle ou éthyle, et E représente un atome d'halogène et leurs sels d'addition avec les acides pharmaceutiquement acceptables ainsi que ceux répondant à la formule (I) dans laquelle A et B ensemble représentent une liaison, G représente un radical alkylthio et D représente un radical phényle non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone et leurs sels d'addition avec les acides pharmaceutiquement acceptables.

Parmi les médicaments préférés de l'invention, on retient tout particulièrement les dérivés répondant à la formule (I) ci-dessus dont les noms suivent :

- la 1-(3-bromo-6-éthyl-5-méthyl-7-méthylthioimidazo [1,2-a] pyrimidin-2-yl) 2-méthyl 2-propèn-1-one ;
- la 1-[5-bromo-1-(4-éthylphényl)-2-méthylthioimidazol-4-yl] 2-méthyl 2-propèn-1-one ;
- la 1-[5-bromo-1-(4-méthoxyphényl)-2-méthylthioimidazol-4-yl] 2-propèn-1-one ;
- la 1-(3-bromo-6-éthyl-7-méthoxy-5-méthylimidazo [1,2-a] pyrimidin-2-yl) 2-propèn-1-one ; et
- la 1-(3-bromo-6-éthyl-7-méthoxy-5-méthylimidazo [1,2-a] pyrimidin-2-yl) 3-phényl 2-propyn-1-one ;

et leurs sels d'addition avec les acides pharmaceutiquement acceptables.

Ces médicaments trouvent, par exemple, leur emploi dans le traitement des maladies inflammatoires et des troubles du système immunorégulateur tels que l'arthrite rhumatoïde, l'ostéoarthrite et le psoriasis.

La dose usuelle, variable selon le produit utilisé, le sujet traité et l'affection en cause peut être par exemple de 0,1 mg à 200 mg par jour, par voie orale, chez l'adulte.

L'invention a également pour objet les compositions pharmaceutiques qui renferment au moins un dérivé précité ou l'un de ses sels d'addition avec les acides pharmaceutiquement acceptables à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les capsules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les 4-imidazoles carboxaldéhydes de formule (II) peuvent être préparés selon une variante des procédés décrits dans Helv. Chim. Acta., (1960), 43, 1787 et dans Tetrahedron (1963), 19, 1883.

Un exemple est donné dans la préparation A ci-dessous.

Les imidazo [1,2-a] pyrimidin-2-carboxaldéhydes de formule (II) peuvent être préparés comme indiqué dans la demande de brevet anglais GB-A-2 128 989 ou par des procédés analogues, comme peuvent l'être également les composés de formule ($I_A$) dans laquelle A′ et B′ ensemble représentent une liaison, G′ et D′ ensemble représente un groupe de formule

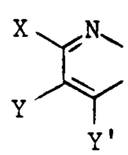

dans laquelle X, Y et Y′ sont définis comme précédemment, et $R'_1$ représente un radical

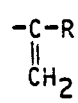

dans lequel R est défini comme précédemment, composés qui bien que n'étant pas eux-mêmes des produits selon l'invention sont toutefois des intermédiaires utiles dans la préparation des composés de formule ($I_B$). Un exemple d'une telle préparation est donnée à la préparation du produit H ci-dessous.

Les imidazo [1,2-a] pyridine-2-carboxaldéhydes de formule (II) peuvent être préparés selon une variante du procédé décrit dans la demande de brevet anglais GB-A-2 128 989 à partir des imidazo [1,2-a] pyridine-2-carboxylates correspondants, qui peuvent être eux-mêmes obtenus selon le procédé décrit dans J. Org. Chem. (1965), 30, 2403.

Les exemples suivants illustrent l'invention sans toutefois la limiter.

Préparation du produit A : 1 – (4 – méthoxyphényl) 2 – méthylthioimidazol – 4 – carboxaldéhyde.

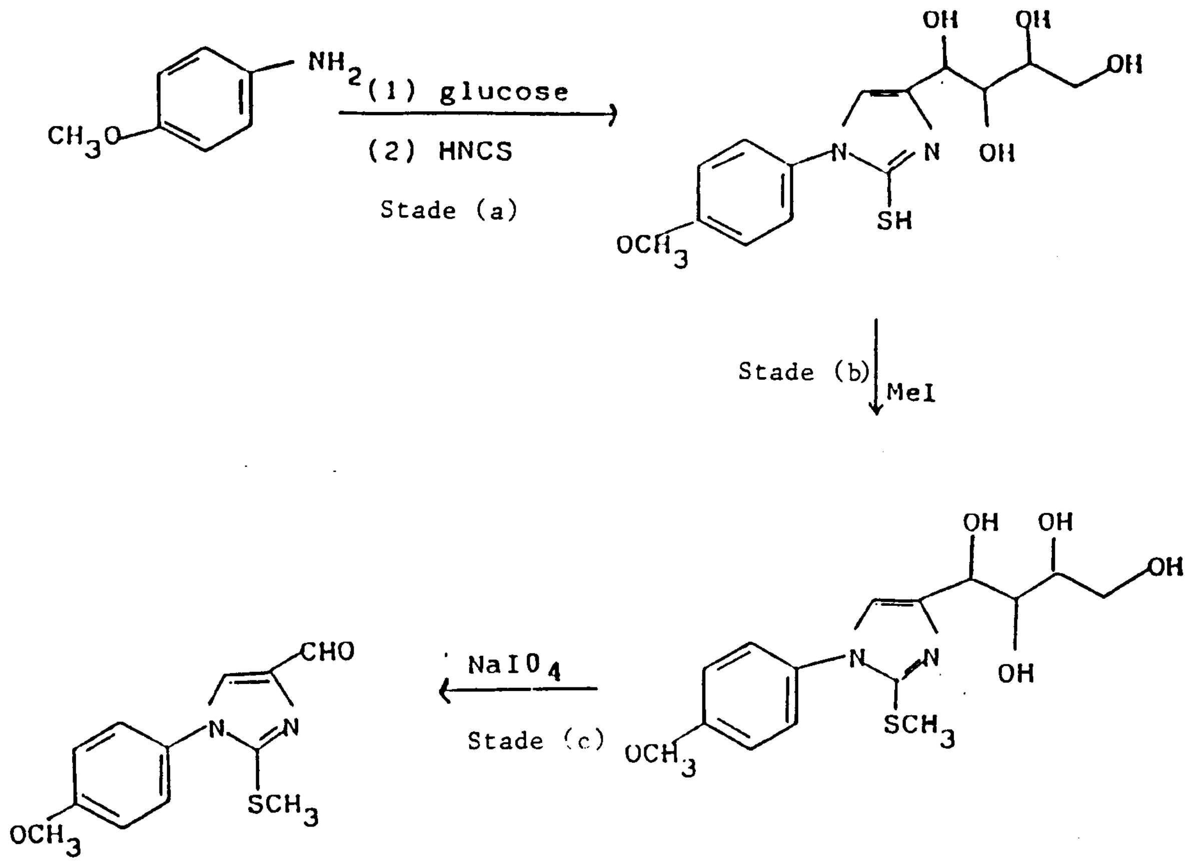


Stade a :

On chauffe pendant 15 minutes au bain – marie bouillant une solution comprenant 18 g de glucose, 12,3 g de 4 – méthoxyaniline, 36 cm3 d'eau et 1 cm3 d'acide acétique. On ajoute 7,6 g d'isothiocyanate d'ammonium et 6 g d'acide acétique et poursuit le chauffage dans les mêmes conditions pendant 2 heures. On dilue le solide obtenu dans l'eau chaude, filtre, agite dans du méthanol pendant 1 heure, filtre, lave à l'éther, sèche et obtient 10,5 g de produit attendu. F = 215 – 216¤C. (Tetrahedron, (1963), 19, 1883. F = 215 – 216¤C.

Stade b :

On mélange pendant 1 heure, 10,5 g du produit obtenu précédemment, 5 g d'iodométhane et 5 g de carbonate de potassium dans 50 cm3 de diméthyl formamide, dilue dans l'eau et recueille 7,6 g de produit attendu cristallisé utilisé tel quel au stade suivant.

Stade c :

On mélange pendant 1 heure, 7,6 g du produit obtenu précédemment et 16 g de métapériodate de sodium dans 100 cm3 d'une solution aqueuse de méthanol à 10%. On concentre sous pression réduite, dilue à l'eau et recueille 2,7 g de produit attendu, après cristallisation dans l'éthanol. F = 118 – 120¤C.

- Les produits B à E ont été préparés de manière analogue.
- Le produit F a été préparé comme indiqué dans la demande de brevet GB – A – 2 128 989 et le produit G a été préparé selon une méthode analogue.

Préparation du produit H : 1-(6-éthyl 5-méthyl 7-méthylthioimidazo [1,2-a] pyrimidin-2-yl) 2-méthyl 2-propèn-1-one (méthode A).

Stade a :

On ajoute sous atmosphère inerte 1,2 g de 6-éthyl 5-méthyl 7-méthylthioimidazo [1,2-a] pyrimidine-2-carboxaldéhyde à une solution de 7,17 mmole de bromure de 2-propényl magnésium dans 50 cm3 de tétrahydrofuranne et agite 3 heures à température ambiante. On verse le milieu réactionnel dans une solution aqueuse de chlorure d'ammonium, extrait au chloroforme, sèche la phase organique et élimine les solvants sous pression réduite. On chromatographie le résidu sur silice (éluant : dichlorométhane-acétate d'éthyle de 1-0 à 0-1) et obtient 1,17 g de 2-méthyl 2-propèn-1-ol. F = 116-118¤C.

Spectre IR :

max. (KBr) : 3160 $cm^{-1}$, 2960 $cm^{-1}$, 2920 $cm^{-1}$, 2870 $cm^{-1}$, 1610 $cm^{-1}$.

Stade b :

On chauffe au reflux pendant 2 heures 1,4 g de produit obtenu précédemment en solution dans 200 cm3 de chloroforme et 5 g de dioxyde de manganèse. On filtre à chaud puis élimine les solvants sous pression réduite et obtient 1,25 g du produit attendu après cristallisation dans l'acétate d'éthyle. F = 136-137¤C.

Les produits J et K ont été préparés de manière analogue.

Les rendements, points de fusion et résultats d'analyse des produits de formule (II) sont donnés dans les tableaux A et B ci-après.

Les rendements, points de fusion et résultats d'analyse des produits intermédiaires de formule ($I_A$) sont donnés dans le tableau C ci-après.

**Exemple 1 : 1-(3-bromo 6-éthyl 5-méthyl 7-méthylthioimidazo [1,2-a] pyrimidin-2-yl) 2-méthyl 2-propèn-1-one (Méthode B).**

On ajoute 0,71 g de N-bromosuccinimide dans 1 g de 1-(6-éthyl 5-méthyl 7-méthylthioimidazo [1,2-a] pyrimidin-2-yl) 2-méthyl 2-propèn-1-one (produit H) en solution dans 50 cm3 de chloroforme et agite 20 minutes à température ambiante. On élimine les solvants sous pression réduite, chromatographie le résidu sur silice (éluant : dichlorométhane) et obtient 1,1 g de produit attendu. F = 87-89¤C.

En opérant comme pour la préparation du produit H (Méthode A) ou comme à l'exemple 1 (méthode B), au départ des produits correspondants de formule (II) ou ($I_A$) respectivement, on a préparé les produits des exemples 2 à 13.

**Exemple 2 : (3-bromo 6-éthyl 7-méthoxy 5-méthylimidazo [1,2-a] pyrimidin-2-yl) isopropényl méthanone.**

**Exemple 3 : (3-chloro 6-éthyl 7-méthoxy 5-méthylimidazo [1,2-a] pyrimidin-2-yl) isopropényl méthanone.**

**Exemple 4 : (7-méthoxy 5-méthylimidazo [1,2-a] pyrimidin-2-yl) éthényl méthanone.**

**Exemple 5 : (3-bromo 6-éthyl 7-méthoxy 5-méthylimidazo [1,2-a] pyrimidin-2-yl) éthényl méthanone.**

**Exemple 6 : [1-(4-éthylphényl) 2-imidazol-4-yl] isopropényl méthanone.**

**Exemple 7 : [1-(4-éthylphényl) 5-bromo 2-thiométhylimidazol-4-yl] isopropényl méthanone.**

**Exemple 8 : [1-(4-méthoxyphényl) 5-bromo 2-thiométhylimidazol-4-yl] isopropényl méthanone.**

**Exemple 9 : [1−(4−méthoxyphényl) 5−chloro 2−thiométhylimidazol−4−yl] isopropényl méthanone.**

**Exemple 10 : [1−(4−méthylphényl) 5−bromo 2−thiométhylimidazol−4−yl] isopropényl méthanone.**

**Exemple 11 : [1−(4−méthylphényl) 5−bromo 2−thioisopropylimidazol−4−yl] isopropényl méthanone.**

**Exemple 12 : [1−(4−fluorophényl) 5−bromo 2−thiométhylimidazol−4−yl] isopropényl méthanone.**

**Exemple 13 : [1−(4−méthoxyphényl) 5−bromo 2−thiométhylimidazol−4−yl] éthényl méthanone.**

**Exemple 14 : [5−bromo 1−(4−hydroxyphényl) 2−méthylthioimidazol−4−yl] 2−méthyl 2−propèn−1−one.**

**Stade (a):** 1−(4−benzyloxyphényl) 2−méthylthioimidazol−4−carboxaldéhyde.

En opérant comme indiqué à la préparation du produit A à partir de la 4−benzyloxyaniline, on a obtenu le produit attendu.

**Stade (b) :** [1−(4−benzyloxyphényl) 2−méthylthioimidazol−4−yl] 2−méthyl 2−propèn−1−one.

En opérant comme au stade (a) de la préparation du produit H (méthode A) à partir du produit obtenuau stade (a) précédent, on a obtenu le produit attendu.

**Stade (c) :** [1−(4−benzyloxyphényl) 5−bromo 2−méthylthioimidazol−4−yl] 2−méthyl 2−propèn−1−one.

En opérant comme indiqué à l'exemple 1 (méthode B) à partir du produit obtenu au stade (b) précédent, on a obtenu le produit attendu.

**Stade (d) :** [5−bromo 1−(4−hydroxyphényl) 2−méthylthioimidazol−4−yl] 2−méthyl 2−propèn−1−one.

On chauffe à 30¤C pendant 24 heures 2,7 g de produit obtenu au stade (c) précédent dans 25 cm3 d'acide trifluoroacétique. On élimine le solvant à 30¤C sous pression réduite, ajoute de l'eau glacée, décante, extrait à l'huile, à l'éther et purifie par chromatographie sur silice (éluant : acétate d'éthyle−éther de pétrole 5−95) et obtient 1,9 g de produit attendu. F = 159−161¤C.

**Exemple 15 : (éthyl 5−bromo 2−thioisopropylimidazol−4−ylisopropényl) méthanone.**

En opérant comme indiqué à l'exemple 1 au départ du produit de formule ($I_A$), on a obtenu le produit attendu.

**Exemple 16 : [5−chloro 1−(4−éthylphényl) 2−(prop−2−ylsulfinyl) imidazol−4−yl] 2−méthyl 2−propèn−1−one (méthode C).**

On chauffe à 50¤C pendant 3 heures, 3 g de [5−chloro 1−(4−éthyl phényl) 2−(prop−2−ylthio) imidazol−4−yl] 2−méthyl 2−propèn−1−one et 2,3 g de métapériodate de sodium dans 30 cm3 d'une solution aqueuse de méthanol (90%). On refroidit, verse dans l'eau, extrait au dichlorométhane, chromatographie sur silice l'huile obtenue (éluant : acétate d'éthyleéther de pétrole 20−80) et obtient 1,3 g de produit attendu. F = 83−84¤C.

**Exemple 17 : [1−(4−éthylphényl) 5−chloro 2−éthylsulfinylimidazol−4−yl] isopropényl méthanone.**

En opérant comme àl'exemple 16 (méthode C) au départ du produit de formule ($I_C$) correspondant, on a obtenu le produit attendu.

En opérant comme à la préparation du produit H (méthode A) ou comme à l'exemple 1 (méthode B) à partir des produits correspondants de formule (II) ou ($I_A$) respectivement, on a préparé les produits des exemples 18 à 21.

**Exemple 18 : (6–éthyl 7–méthoxy 5–méthylimidazo [1,2–a] pyrimidin–2–yl) phényléthynyl méthanone.**

**Exemple 19 : 1–(6–éthyl 7–méthoxy 5–méthylimidazo [1,2–a] pyrimidin–2–yl) (3,3–diméthylbut–1–ynyl) méthanone.**

**Exemple 20 : (3–bromo 6–éthyl 7–méthoxy 5–méthylimidazo [1,2–a] pyrimidin–2–yl) (3,3–diméthylbut–1–ynyl) méthanone.**

**Exemple 21 : (3–bromo 6–éthyl 7–méthoxy 5–méthylimidazo [1,2–a] pyrimidin–2–yl) (2–phényléthy–1–yl) méthanone.**

**Exemple 22 : 1–(3–bromo 6–éthyl 7–méthoxy 5–méthylimidazo [1,2–a] pyrimidin–2–yl) 4–hydroxy 2–butyn–1–one.**

**Stade (a) :** 1–(6–éthyl 7–méthoxy 5–méthylimidazo [1,2–a] pyrimidin–2–yl) 4–(tétrahydropyran–2–yloxy) 2–butyn–1–one.

En opérant comme pour la préparation du produit H (méthode A) à partir du 6–éthyl 7–méthoxy 5–méthylimidazo [1,2–a] pyrimidin–2–carboxaldéhyde et du bromure de 3–(tétrahydropyran–2–yloxy) 1–propynyl magnésium préparé comme il est indiqué dans J. Chem. Soc., (1950), 3646, on a obtenu le produit attendu.

**Stade (b) :** 1–(3–bromo 6–éthyl 7–méthoxy 5–méthylimidazo [1,2–a] pyrimidin–2–yl) 4–(tétrahydropyran–2–yloxy) 2–butyn–1–one.

En opérant comme à l'exemple 1 (méthode B) à partir du produit obtenu au stade (a) précédent, on a obtenu le produit attendu.

**Stade (c) :** 1–(3–bromo 6–éthyl 7–méthoxy 5–méthylimidazo [1,2–a] pyrimidin–2–yl) 4–hydroxy 2–butyn–1–one.

On agite 30 minutes à température ambiante 5 g de 1–(3–bromo 6–éthyl 7–méthoxy 5–méthylimidazo [1,2–a] pyrimidin–2–yl) 4–(tétrahydropyran–2–yloxy) 2–butyn–1–one en solution dans 100 cm3 de méthanol et 35 cm3 d'acide chlorhydrique 2 N. On neutralise le milieu réactionnel à l'aide de bicarbonate de sodium, extrait au chloroforme, et après élimination des solvants sous pression réduite, chromatographie le résidu sur silice (éluant : chloroforme–éther–méthanol variant de 100–0–0 à 0–95–5). On obtient 1,3 g de produit attendu. F = 212–213¤C (décomposition).

**Exemple 23 : 1–(6–éthyl 7–méthoxy 5–méthylimidazo [1,2–a] pyrimidin–2–yl) 4–hydroxy 2–butyn–1–one.**

**Stade (a) :** 1–(6–éthyl 7–méthoxy 5–méthylimidazo [1,2–a] pyrimidin–2–yl) 4–(tétrahydropyran–2–yloxy) 2–butyn–1–one.

En opérant comme au stade (a) de l'exemple 22, on a obtenu le produit attendu.

**Stade (b) :** 1–(6–éthyl 7–méthoxy 5–méthylimidazo [1,2–a] pyrimidin–2–yl) 4–hydroxy 2–butyn–1–one.

En opérant comme au stade (c) de l'exemple 22, au départ du produit obtenu au stade (a) précédent, on a obtenu le produit attendu.

En opérant comme indiqué pour la préparation du produit H (méthode A) ou comme à l'exemple 1 (méthode B) en partant des produits correspondants de formule (II) ou ($I_A$) respectivement, on a préparé les

produits des exemples 24 à 28.

**Exemple 24 : (3-bromo 6-éthyl 7-méthoxy 5-méthylimidazo [1,2-a] pyrimidin-2-yl) (propyn-1-ynyl) méthanone.**

**Exemple 25 : (3-bromoimidazo [1,2-a] pyridin-2-yl) isopropényl méthanone (chlorhydrate).**

**Exemple 26 : (imidazo [1,2-a] pyridin-2-yl) isopropényl méthanone.**

**Exemple 27 : (imidazo [1,2-a] pyridin-2-yl) (2-phényléthyn-1-yl) méthanone.**

**Exemple 28 : 3-bromoimidazo [1,2-a] pyridin-2-yl (2-phényléthyn-1-yl) méthanone.**

Les rendements, points de fusion, résultats d'analyse figurent dans les tableaux 1 à 4 ci-après.

TABLEAU A

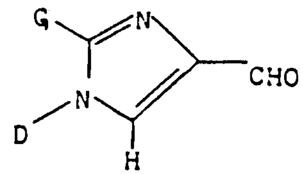

| PREP | G | D | Rendt. (%) | Spectre IR (KBr) ($cm^{-1}$) | F (°C) | Formule | Poids Molé-culaire | Calculé/Trouvé (%) C | H | N |
|---|---|---|---|---|---|---|---|---|---|---|
| A | MeS | 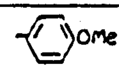 | 49 | 3130,1690,1670 | 118-20 | $C_{12}H_{12}N_2O_2S$ | 248.3 | 58.05<br>57.65 | 4.87<br>4.88 | 11.28<br>11.09 |
| B | MeS | 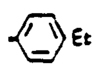 | 80 | 3130,1668,1650 | 88-90 | $C_{13}H_{14}N_2OS$ | 246.3 | 63.39<br>63.44 | 5.73<br>5.73 | 11.37<br>11.41 |
| C | MeS | 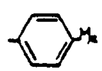 | 83 | 1680 | 111-3 | $C_{12}H_{12}N_2OS$ | 232.3 | | | |
| D | iPrS | 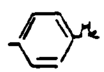 | 75 | 1670 | 63-4 | $C_{14}H_{16}N_2OS$ | 260.3 | | | |
| E | MeS | 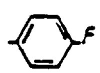 | 90 | 1690,1675 | 130-1 | $C_{11}H_9FN_2OS$ | 236.3 | | | |

TABLEAU B

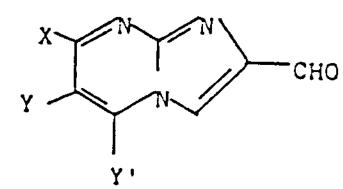

| PREP | X | Y | Y' | Rendt. (%) | Spectre IR (KBr) ($cm^{-1}$) | F (°C) | Formule | Poids Molé-culaire | Calculé/Trouvé (%) | | | |
|---|---|---|---|---|---|---|---|---|---|---|---|---|
| | | | | | | | | | C | H | N | S |
| F | MeO | Et | Me | 72 | 3120,3000,2980, 2950,1690,1640 | 185-6 | $C_{11}H_{13}N_3O_2$ | 219.2 | 60.26<br>59.97 | 5.98<br>5.97 | 19.17<br>19.05 | |
| G | MeS | Et | Me | 85 | 3098,2960,2915, 2860,1675,1610 | 203-4 | $C_{11}H_{13}N_3OS$ | 235.3 | 56.15<br>56.35 | 5.58<br>5.58 | 17.85<br>17.95 | 13.63<br>13.34 |

TABLEAU C

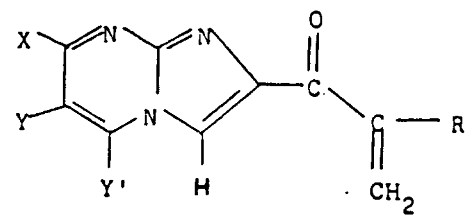

| PREP | X | Y | Y' | R | Rendt. (%) | Spectre IR (KBr) ($cm^{-1}$) | F (°C) | Formule | Poids Molé-culaire | Calculé/Trouvé (%) C | H | N | S |
|---|---|---|---|---|---|---|---|---|---|---|---|---|---|
| H | MeS | Et | Me | Me | 76 | 2970,1630,1615 | 136-7 | $C_{14}H_{17}N_3OS$ | 275.4 | 61.06<br>60.91 | 6.24<br>6.22 | 15.25<br>15.17 | 11.64<br>11.54 |
| J | MeO | Et | Me | Me | 35 | 3130,1640 | 148-50 | $C_{14}H_{17}N_3O_2$ | 259.3 | 64.85<br>64.67 | 6.61<br>6.57 | 16.20<br>16.12 | |
| K | MeO | H | Me | Me | 56 | 3150,2980,2950,<br>1625 | 151 | $C_{12}H_{13}N_3O_2$ | 231.25 | 62.32<br>62.20 | 5.68<br>5.70 | 18.16<br>18.14 | |

TABLEAU 1

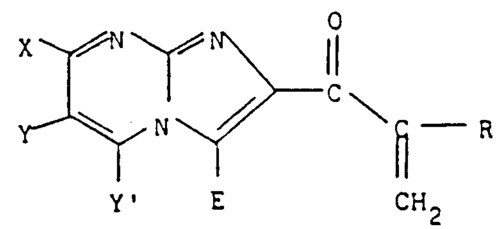

| Ex | X | Y | Y' | E | R | Methode | Rendt (%) | Spectre IR (KBr) ($cm^{-1}$) | F (°C) | Formule | Poids Molé-culaire | Calculé/Trouvé(%) | | | | |
|---|---|---|---|---|---|---|---|---|---|---|---|---|---|---|---|---|
| | | | | | | | | | | | | C | H | N | Hal | S |
| 1 | MeS | Et | Me | Br | Me | B | 87 | 2960, 2920, 1640, 1600 | 87-9 | $C_{14}H_{16}BrN_3OS$ | 354.3 | 47.46<br>47.32 | 4.56<br>4.56 | 11.86<br>11.82 | 22.55(Br)<br>22.54 | 9.05<br>9.02 |
| 2 | MeO | Et | Me | Br | Me | B | 87 | 2960, 1635, 1625 | 101-2 | $C_{14}H_{16}BrN_3O_2$ | 338.2 | 49.71<br>49.73 | 4.78<br>4.72 | 12.42<br>12.31 | 23.63(Br)<br>23.67 | |
| 3 | MeO | Et | Me | Cl | Me | B | 62 | 2965, 1625 | 111-2 | $C_{14}H_{16}ClN_3O_2$ | 293.8 | 57.24<br>57.03 | 5.50<br>5.49 | 14.30<br>14.25 | 12.07(Cl)<br>12.17 | |
| 4 | MeO | H | Me | Br | Me | B | 98 | 3070, 2950, 1630 | 145-6 decomp | $C_{12}H_{12}BrN_3O_2$ | 310.15 | – | | | | |
| 5 | MeO | Et | Me | Br | H | B | 47 | 2970, 2940, 2875, 1670, 1625 | 120-3 decomp | $C_{13}H_{14}BrN_3O_2$ | 324.2 | 48.16<br>48.05 | 4.36<br>4.32 | 12.96<br>12.73 | 24.65(Br)<br>24.45 | |

TABLEAU 2

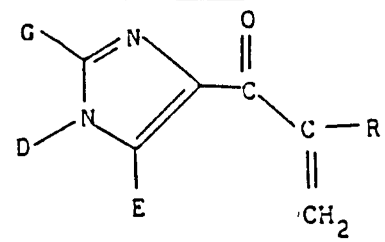

| Exemple | G | D | E | R | Methode | Rendt (%) | Spectre IR (KBr) ($cm^{-1}$) | F (°C) | Formule | Poids Molé-culaire | Calculé/Trouvé (%) | | | |
|---|---|---|---|---|---|---|---|---|---|---|---|---|---|---|
| | | | | | | | | | | | C | H | N | S |
| 6 | MeS | 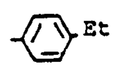 | H | Me | A | 43 | 3120,1636, 1626,1611 | 46-7 | $C_{16}H_{18}N_2OS$ | 286.4 | 67.10 67.18 | 6.33 6.24 | 9.78 9.77 | 11.19 11.23 |
| 7 | MeS | 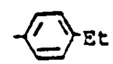 | Br | Me | B | 77 | 1636,1620 | 94-6 | $C_{16}H_{17}BrN_2OS$ | 365.3 | 52.61 52.62 | 4.69 4.69 | 7.67 7.67 | |

TABLEAU 2 (suite)

| Exemple | G | D | E | R | Methode | Rendt (%) | Spectre IR (KBr) ($cm^{-1}$) | F (°C) | Formule | Poids Molé-culaire | Calculé/Trouvé (%) C | H | N | Other |
|---|---|---|---|---|---|---|---|---|---|---|---|---|---|---|
| 8 | MeS | 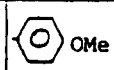 | Br | $CH_3$ | B | 84 | 1630,1620, 1605 | 128-9 | $C_{15}H_{15}BrN_2O_2S$ | 367.3 | 49.05<br>48.86 | 4.12<br>4.10 | 7.63<br>7.55 | 21.76 (Br)<br>21.74 |
| 9 | MeS | 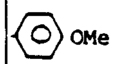 | Cl | $CH_3$ | B | 93 | 1630,1620, 1600 | 110-1 | $C_{15}H_{15}ClN_2O_2S$ | 322.8 | 55.81<br>55.12 | 4.68<br>4.74 | 8.68<br>8.53 | 10.98 (Cl)<br>11.39 |
| 10 | MeS | 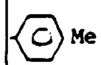 | Br | $CH_3$ | B | 52 | 1630,1610 | 89-90 | $C_{15}H_{15}BrN_2OS$ | 351.3 | 51.29<br>51.09 | 4.30<br>4.29 | 7.98<br>7.89 | 22.75 (Br)<br>22.83 |
| 11 | iPrS | 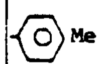 | Br | $CH_3$ | B | 66 | 1635,1615, 1605 | 135-6 | $C_{17}H_{19}BrN_2OS$ | 379.3 | 53.83<br>53.76 | 5.05<br>5.07 | 7.39<br>7.33 | 8.45 (S)<br>8.43 |
| 12 | MeS | 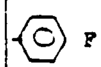 | Br | $CH_3$ | B | 75 | 1635,1595 | 99-101 | $C_{14}H_{12}BrFN_2OS$ | 355.2 | 47.33<br>47.37 | 3.41<br>3.48 | 7.81<br>7.81 | 9.02 (S)<br>9.05 |
| 13 | MeS | 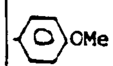 | Br | H | B | 68 | 1655,1600 | 117-9 | $C_{14}H_{13}BrN_2O_2S$ | 353.2 | 47.60<br>47.36 | 3.71<br>3.75 | 7.93<br>7.79 | 22.62 (Br)<br>22.72 |
| 14 | MeS | 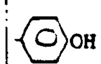 | Br | $CH_3$ | B | 86 | 3220,1610, 1590 | 159-61 | $C_{14}H_{13}BrN_2O_2S$ | 353.2 | 47.60<br>47.63 | 3.71<br>3.72 | 7.93<br>7.82 | |
| 15 | iPrS | Et | Br | $CH_3$ | B | 53 | 2960,1635, 1590 | oil | $C_{12}H_{17}BrNO_2S$ | 317.2 | 45.43<br>44.98 | 5.40<br>5.37 | 8.83<br>8.69 | |
| 16 | iPrSO | 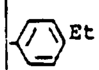 | Cl | $CH_3$ | C | 41 | 1640,1610, 1595 | 83-4 | $C_{18}H_{21}ClN_2O_2S$ | 364.8 | | | | |
| 17 | EtSO | 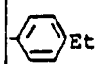 | Cl | $CH_3$ | C | 33 | 1640,1610, 1600 | 76-8 | $C_{17}H_{19}ClN_2O_2S$ | 350.8 | | | | |

TABLEAU 3

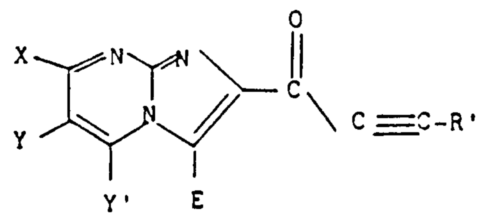

| Ex | X | Y | Y' | E | R' | Methode | Rendt (%) | Spectre IR (KBr) ($cm^{-1}$) | F (°C) | Formule | Poids Moléculaire | Calculé/Trouvé (%) C | H | N | Hal |
|---|---|---|---|---|---|---|---|---|---|---|---|---|---|---|---|
| 18 | MeO | Et | Me | H | Ph | A | 86 | 2197,1630 | 194-6 | $C_{19}H_{17}N_3O_2$ | 319.4 | 71.46<br>71.33 | 5.37<br>5.42 | 13.16<br>13.11 | |
| 19 | MeO | Et | Me | H | t-Bu | A | 71 | 2965,2215,1635 | 133-5 | $C_{17}H_{21}N_3O_2$ | 299.3 | 68.21<br>68.19 | 7.07<br>7.09 | 14.04<br>14.06 | |
| 20 | MeO | Et | Me | Br | t-Bu | B | 86 | 2970,2930,2870,<br>2200,1640,1620 | 186-7 | $C_{17}H_{20}BrN_3O_2$ | 378.3 | 53.97<br>53.72 | 5.34<br>5.33 | 11.10<br>10.97 | 21.12 (Br)<br>20.89 |
| 21 | MeO | Et | Me | Br | Ph | B | 79 | 3050,2960,2920,<br>2200,1630 | 187-<br>190 | $C_{19}H_{16}BrN_3O_2$ | 398.3 | 57.30<br>57.24 | 4.06<br>4.09 | 10.55<br>10.45 | 20.06 (Br)<br>20.11 |
| 22 | MeO | Et | Me | H | $-CH_2OH$ | A | 41 | 3400,3125,2210,<br>1635 | 176-<br>186<br>(decomp) | $C_{14}H_{15}N_3O_3$ | 273.3 | 61.53 | 5.53 | 15.38 | |
| 23 | MeO | Et | Me | Br | $-CH_2OH$ | B | 28 | 3250,2980,2880,<br>2220,1630 | 212-3<br>(decomp) | $C_{14}H_{14}BrN_3O_3$ | 352.2 | 47.74<br>47.63 | 4.01<br>4.08 | 11.93<br>11.78 | 22.69 (Br)<br>22.36 |
| 24 | MeO | Et | Me | Br | $CH_3$ | B | 93 | 2960,2230,2205,<br>1630 | 170-2<br>(decomp) | $C_{14}H_{14}BrN_3O$ | 320.2 | 52.51 | 4.42 | 13.12 | 24.96 (Br) |

TABLEAU 4

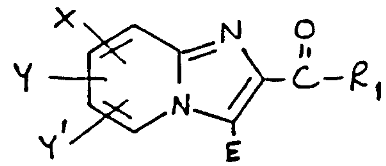

| Ex | X | Y | Y' | E | $R_1$ | Methode | Rendt (%) | Spectre IR (KBr) ($cm^{-1}$) | F (°C) | Formule | Poids Molé-culaire | Calculé/Trouvé(%) | | | |
|---|---|---|---|---|---|---|---|---|---|---|---|---|---|---|---|
| | | | | | | | | | | | | C | H | N | Hal |
| 25 | H | H | H | Br | $-CMe=CH_2$ | B | 79 | 3070,2540,1650 | 140-2 (HCl salt) | $C_{11}H_9BrN_2O$ | 301.6 | 43.81<br>43.57 | 3.35<br>3.41 | 9.28<br>9.11 | |
| 26 | H | H | H | H | $-CMe=CH_2$ | A | 77 | 3145,3100,3080,<br>3040,1635 | 93-4 | $C_{11}H_{10}N_2O$ | 186.2 | 70.95<br>71.08 | 5.42<br>5.40 | 15.04<br>15.03 | |
| 27 | H | H | H | H | -C≡C-Ph | A | 80 | 3140,3100,3080,<br>3050,2190,1630 | 168 | $C_{16}H_{10}N_2O$ | 246.3 | 78.03<br>77.96 | 4.10<br>4.23 | 11.37<br>11.31 | |
| 28 | H | H | H | Br | -C≡C-Ph | B | 74 | 3080,3050,2200,<br>1630 | 133-4 | $C_{16}H_9BrN_2O$ | 325.15 | 59.10<br>59.01 | 2.80<br>2.92 | 8.61<br>8.60 | 24.57(Br)<br>24.66 |

**Exemple 29 :**

On a préparé des comprimés répondant à la formulation suivante :

| | |
|---|---|
| − Produit de l'exemple 1<br>− Excipient q.s. pour un comprimé terminé à | 20 mg<br>100 mg. |

(Détail de l'excipient : lactose, amidon, talc, stéarate de magnésium).

**Exemple 30 :**

On a préparé des comprimés répondant à la formulation suivante :

| | |
|---|---|
| − Produit de l'exemple 7<br>− Excipient q.s. pour un comprimé terminé à | 20 mg<br>100 mg. |

(Détail de l'excipient : lactose, amidon, talc, stéarate de magnésium).

**ETUDE BIOCHIMIOUE**

L'inhibition de l'ionophore $Ca^{++}$ (A 23187) provoque la libération de produits 5−lipoxygénase (Leukotriène $B_4$ et 5−HETE) de l'acide [14c] arachidonique des neutrophiles péritonéales pré−marqués du rat.

On a utilisé la méthode de Ahnfelt−Ronne, I. et Arrigoni−Martelli, E. Biochemical Pharmacology, Vol. 31, n¤ 16, p. 2619−2624 (1982) modifiée. Les valeurs indiquées sont des concentrations micromolaires du composé testé provoquant 50% d'inhibition par rapport à la réponse du témoin déterminées graphiquement d'après les courbes de réponse par dose.

| Exemple | $IC_{50}$ - µM |
|---|---|
| 1 | 6,8 |
| 2 | 17 |
| 3 | 21 |
| 4 | 7,4 |
| 5 | 1,7 |
| 6 | 3,8 |
| 7 | 2,5 |
| 8 | 7,2 |
| 9 | 9,1 |
| 10 | 10 |
| 11 | 3,7 |
| 12 | 15 |
| 13 | 2,3 |
| 14 | > 10 |
| 15 | 17 |
| 16 | 4,4 |
| 17 | - |
| 18 | 12 |
| 19 | 16 |
| 20 | 4,2 |
| 21 | 1,5 |
| 22 | 59 |
| 23 | 46 |
| 24 | 7,2 |
| 25 | 28 |
| 26 | > 100 |
| 27 | 41 |
| 28 | 5,2 |

## Revendications

1. Nouveaux dérivés de l'imidazole et leurs sels d'addition avec les acides, caractérisés en ce qu'ils répondent à la formule (I) :

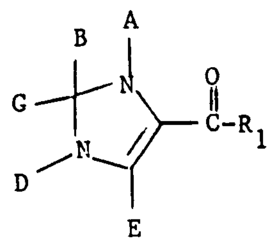

(I)

dans laquelle $R_1$ représente soit un radical

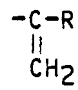

dans lequel R représente un atome d'hydrogène ou un radical alcoyle renfermant de 1 à 5 atomes de carbone, soit un radical $-C{\equiv}C-R'$ dans lequel $R'$ représente un atome d'hydrogène, un radical alcoyle renfermant de 1 à 5 atomes de carbone, un radical hydroxyalcoyle renfermant de 1 à 5 atomes de carbone ou un radical aryle renfermant de 6 à 10 atomes de carbone non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone, et

- soit A représente un radical alcoyle renfermant de 1 à 5 atomes de carbone et B et G ensemble avec l'atome de carbone auquel ils sont liés représentent un radical carbonyle ou thiocarbonyle et D représente un radical alcoyle renfermant de 1 à 5 atomes de carbone ou un radical phényle non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone,
- soit A et B représentent ensemble une liaison et G représente un radical alcoyle, alcoxy, alkylthio, alkylsulfinyle ou alkylsulfonyle renfermant de 1 à 5 atomes de carbone, D représente un radical alcoyle renfermant de 1 à 5 atomes de carbone ou un radical phényle non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone,
- soit A et B représentent ensemble une liaison et G et D ensemble représentent un groupe de formule :

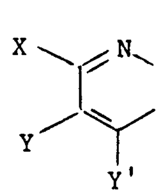

ou

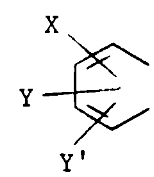

dans laquelle X représente un radical alcoxy ou alkylthio et Y et $Y'$, identiques ou différents, représentent un atome d'hydrogène ou un radical alcoyle renfermant de 1 à 5 atomes de carbone, E représente un atome d'hydrogène ou d'halogène, étant entendu que lorsque A et B ensemble représentent une liaison et G et D ensemble représentent un groupe

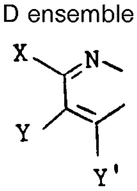

E ne peut pas représenter un atome d'hydrogène si $R_1$ représente un radical

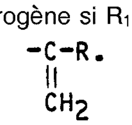

2. Dérivés répondant à la formule (I) de la revendication 1, ainsi que leurs sels, caractérisés en ce que dans ladite formule (I), $R_1$ représente un radical

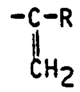

dans lequel R est défini comme à la revendication 1 ou un radical $-C{\equiv}C-R'$ dans lequel R′ est défini comme à la revendication 1 et E représente un atome d'halogène.

3. Dérivés répondant à la formule (I) de la revendication 1 ou 2 ainsi que leurs sels, caratérisés en ce que dans ladite formule (I), A et B ensemble représenent une liaison et G et D ensemble représentent un groupe de formule :

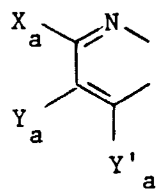

ou

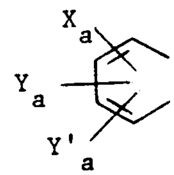

dans laquelle $X_a$ représente un radical méthoxy ou méthylthio et $Y_a$ et $Y'_a$, identiques ou différents, représentent un atome d'hydrogène ou un radical méthyle ou éthyle, et E représente un atome d'halogène.

4. Dérivés répondant à la formule (I) de la revendication 1 ou 2 ainsi que leurs sels, caractérisés en ce que dans ladite formule (I), A et B ensemble représentent une liaison, G représente un radical alkylthio et D représente un radical phényle non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone.

5. Dérivés répondant à la formule (I) de la revndication 1 dont les noms suivent :
   - la 1-(3-bromo-6-éthyl-5-méthyl-7-méthylthioimidazo [1,2-a] pyrimidin-2-yl) 2-méthyl 2-propèn-1-one ;
   - la 1-[5-bromo-1-(4-éthylphényl)-2-méthylthioimidazol-4-yl] 2-méthyl 2-propèn-1-one ;
   - la 1-[5-bromo-1-(4-méthoxyphényl)-2-méthylthioimidazol-4-yl] 2-propèn-1-one ;
   - la 1-(3-bromo-6-éthyl-7-méthoxy-5-méthylimidazo [1,2-a] pyrimidin-2-yl) 2-propèn-1-one ; et
   - la 1-(3-bromo-6-éthyl-7-méthoxy-5-méthylimidazo [1,2-a] pyrimidin-2-yl) 3-phényl 2-propyn-1-one ;

   et leurs sels d'addition avec les acides.

6. Procédé de préparation des produits tels que définis par la formule (I) de la revendication 1, répondant à la formule :

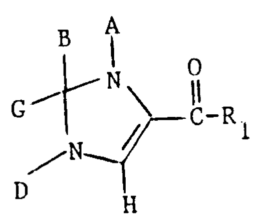

dans laquelle A, B, G, D et $R_1$ dont définis comme précédemment ainsi que de leurs sels, caractérisés en ce que l'on soumet un produit de formule (II) :

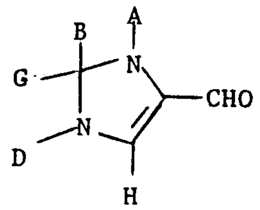

(II)

dans laquelle A, B, G et D sont définis comme précédemment à un générateur de carbanions $R_1^-$ tel qu'un réactif de Grignard de formule (III) :

$R_1 - Mg - Br$ (III)

ou un alkyllithien de formule ($III_A$) :

$R_1 - Li$ ($III_A$)

formules dans lesquelles $R_1$ est défini comme précédemment pour obtenir un produit de formule (IV) :

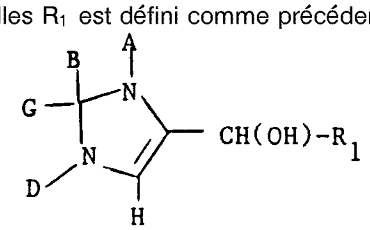

(IV)

dans laquelle A, B, G, D et $R_1$ sont définis comme précédemment que l'on soumet à une oxydation pour obtenir le produit de formule générale (I) dans laquelle E représente un atome d'hydrogène que, le cas échéant, l'on salifie.

**7.** Procédé de préparation des produits tels que définis par la formule (I) de la revendication 1, répondant à la formule ($I_B$) :

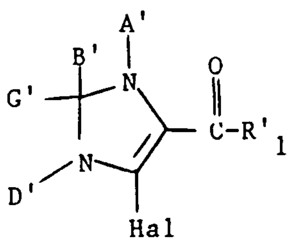

($I_B$)

dans laquelle $R'_1$ représente un radical

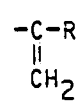

dans lequel R est défini comme précédemment ou un radical $-C \equiv C - R'$ dans lequel R′ est défini comme précédemment et

- soit A′ représente un radical alcoyle renfermant de 1 à 5 atomes de carbone et B′ et G′ ensemble avec l'atome de carbone auquel ils sont liés représentent un radical carbonyle ou thiocarbonyle et D′ représente un radical alcoyle renfermant de 1 à 5 atomes de carbone ou un radical phényle non substitué ou substitué par un ou plusieurs substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone,
- soit A′ et B′ représentent ensemble une liaison et G′ représente un radical alcoyle ou alkylthio, renfermant de 1 à 5 atomes de carbone, et D′ représente un radical alcoyle renfermant de 1 à 5 atomes de carbone ou un radical phényle non substitué ou substitué par un ou plusieurs

substituants choisis parmi les atomes d'halogène, les radicaux hydroxy, carboxy, trifluorométhyle, alcoyle renfermant de 1 à 5 atomes de carbone et alcoxy renfermant de 1 à 5 atomes de carbone,

- soit A′ et B′ représentent ensemble une liaison et G′ et D′ ensemble représentent un groupe de formule :

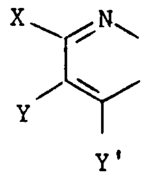

ou

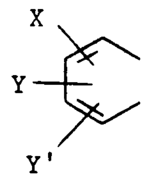

dans laquelle X, Y et Y′ sont définis comme précédemment et Hal représente un atome d'halogène, ainsi que de leurs sels, caractérisé en ce que l'on traite un produit de formule ($I_A$) :

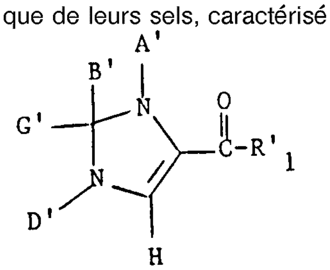

($I_A$)

dans laquelle A′, B′, G′, D′ et $R'_1$ sont définis comme ci-dessus par un N-halosuccinimide pour obtenir un produit de formule ($I_B$) que, le cas échéant, l'on salifie.

8. Procédé de préparation des produits tels que définis par la formule (I) de la revendication 1, répondant à la formule ($I_D$) :

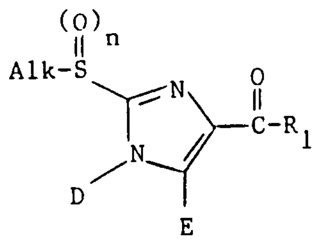

($I_D$)

dans laquelle $R_1$, D et E sont définis comme précédemment, Alk représente un radical alcoyle renfermant de 1 à 5 atomes de carbone et n a la valeur 1 ou 2 ainsi que de leurs sels, caractérisé en ce que l'on oxyde un produit de formule ($I_C$) :

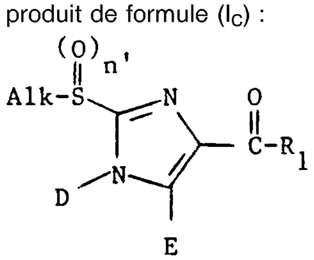

($I_C$)

dans laquelle $R_1$, D, E et Alk sont définis comme ci-dessus et n′ a la valeur 0 ou 1 pour obtenir un produit de formule ($I_D$) que, le cas échéant, l'on salifie.

9. Procédé de préparation des produits tels que définis par la formule (I) de la revendication 1 dans laquelle l'un au moins des substituants $R_1$ et D comprend un radical hydroxy, caractérisé en ce que l'on déprotège le produit correspondant de formule (V) :

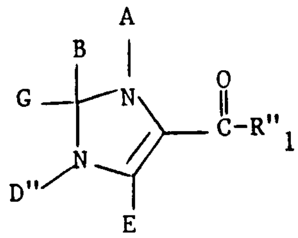

(V)

dans laquelle A, B, G et E sont définis comme précédemment, $D''$ a soit la signification indiquée pour D, soit lorsque l'on veut obtenir un produit de formule (I) dans laquelle D représente un radical hydroxy libre, $D''$ représente un précurseur de D contenant un radical hydroxy protégé et $R''_1$ a soit la signification indiquée pour $R_1$, soit lorsque l'on veut obtenir un produit de formule (I) dans laquelle $R_1$ représente un radical hydroxy libre, $R''_1$ représente un précurseur de $R_1$ contenant un radical hydroxy protégé, étant entendu que l'un au moins de $D''$ et de $R''$ représente respectivement un précurseur de D et de $R_1$ contenant un radical hydroxy protégé pour obtenir un produit de formule (I) dans laquelle $R_1$ et D sont définis comme ci-dessus que, le cas échéant, l'on salifie.

**10.** Procédé de préparation selon l'une quelconque des revendications 6 à 9, caractérisé en ce que :
- l'oxydation des produits de formule (IV) est effectuée de préférence par le dioxyde de manganèse ;
- l'oxydation des produits de formule ($I_C$) est effectuée de préférence par le métapériodate de sodium.

**11.** Médicaments, caractérisés en ce qu'ils sont constitués par les nouveaux dérivés de l'imidazole tels que définis à la revendication 1, ainsi que par leurs sels d'addition avec les acides pharmaceutiquement acceptables.

**12.** Médicaments, caractérisés en ce qu'ils sont constitués par les nouveaux dérivés de l'imidazole tels que définis à l'une quelconque des revendications 2, 3 ou 4, ainsi que par leurs sels d'addition avec les acides pharmaceutiquement acceptables.

**13.** Médicaments, caractérisés en ce qu'ils sont constitués par les nouveaux dérivés de l'imidazole tels que définis à la revendication 5, ainsi que par leurs sels d'addition avec les acides pharmaceutiquement acceptables.

**14.** Compositions pharmaceutiques, caractérisées en ce qu'elles renferment à titre de principe actif, l'un au moins des médicaments tels que définis à l'une quelconque des revendications 11, 12 et 13.

## Claims

**1.** New imidazole derivatives and their addition salts with acids, characterized in that they correspond to formula (I):

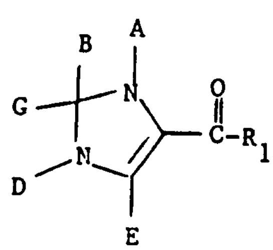

(I)

in which $R_1$ represents either a

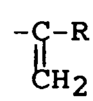

radical in which R represents a hydrogen atom or an alkyl radical containing 1 to 5 carbon atoms, or a −C≡C−R' radical in which R' represents a hydrogen atom, an alkyl radical containing 1 to 5 carbon atoms, a hydroxyalkyl radical containing 1 to 5 carbon atoms or an aryl radical containing 6 to 10 carbon atoms, non−substituted or substituted by one or more substituents chosen from halogen atoms, the following radicals: hydroxy, carboxy, trifluoromethyl, alkyl containing 1 to 5 carbon atoms and alkoxy containing 1 to 5 carbon atoms, and

- either A represents an alkyl radical containing 1 to 5 carbon atoms and B and G together with the carbon atom to which they are linked represent a carbonyl or thiocarbonyl radical and D represents an alkyl radical containing 1 to 5 carbon atoms or a phenyl radical non−substituted or substituted by by one or more substituents chosen from halogen atoms, the following radicals: hydroxy, carboxy, trifluoromethyl, alkyl containing 1 to 5 carbon atoms and alkoxy containing 1 to 5 carbon atoms,
- or A and B together represent a bond and G represents an alkyl, alkoxy, alkylthio, alkylsulphinyl or alkylsulphonyl radical containing 1 to 5 carbon atoms, D represents an alkyl radical containing 1 to 5 carbon atoms or a phenyl radical non−substituted or substituted by one or more substituents chosen from halogen atoms, the following radicals: hydroxy, carboxy, trifluoromethyl, alkyl containing 1 to 5 carbon atoms and alkoxy containing 1 to 5 carbon atoms;
- or A and B together represent a bond and G and D together represent a group of formula:

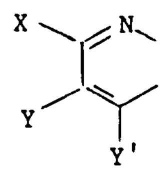

or

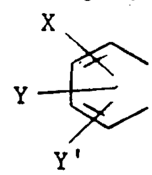

in which X represents an alkoxy or alkylthio radical and Y and Y', identical or different, represent a hydrogen atom or an alkyl radical containing 1 to 5 carbon atoms, E represents a hydrogen or halogen atom, it being understood that when A and B together represent a bond and G and D together represent a group

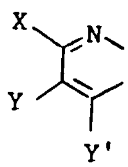

E cannot represent a hydrogen atom if $R_1$ represents a radical.

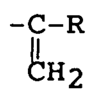

2. Derivatives corresponding to formula (I) of claim 1, as well as their salts, characterized in that in the said formula (I), $R_1$ represents a

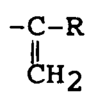

radical in which R is defined as in claim 1 or a −C = C − R' radical in which R' is defined as in claim 1 and E represents a halogen atom.

3. Derivatives corresponding to formula (I) of claim 1 or 2 as well as their salts, characterized in that in the said formula (I), A and B together represent a bond and G and D together represent a group of formula:

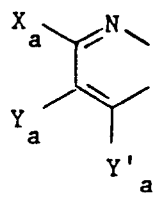 or 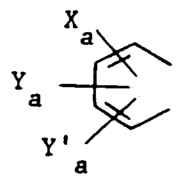

in which $X_a$ represents a methoxy or methylthio radical and $Y_a$ and $Y'_a$, identical or different, represent a hydrogen atom or a methyl or ethyl radical, and E represents a halogen atom.

**4.** Derivatives corresponding to formula (I) of claim 1 or 2 as well as their salts, characterized in that in the said formula (I), A and B together represent a bond, G represents an alkylthio radical and D represents a phenyl radical, non-substituted or substituted by one or more substituents chosen from halogen atoms, the following radicals: hydroxy, carboxy, trifluoromethyl, alkyl containing 1 to 5 carbon atoms and alkoxy containing 1 to 5 carbon atoms.

**5.** Derivatives corresponding to formula (I) of claim 1 of which the names follow:
- 1-(3-bromo-6-ethyl-5-methyl-7-methylthioimidazo [1,2-a] pyrimidin-2-yl) 2-methyl 2-propen-1-one;
- 1-[5-bromo-1-(4-ethylphenyl)-2-methylthioimidazol-4-yl] 2-methyl 2-propen-1-one;
- 1-[5-bromo-1-(4-methoxyphenyl)-2-methylthioimidazol-4-yl] 2-propen-1-one;
- 1-(3-bromo-6-ethyl-7-methoxy-5-methylimidazo [1,2-a] pyrimidin-2-yl) 2-propen-1-one; and
- 1-(3-bromo-6-ethyl-7-methoxy-5-methylimidazo [1,2-a] pyrimidin-2-yl) 3-phenyl 2-propyn-1-one;

and their addition salts with acids.

**6.** Preparation process for the products as defined by formula (I) of claim 1, corresponding to formula:

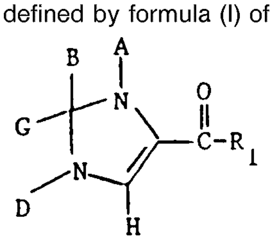

in which A, B, G, D and $R_1$ are defined as previously, as well as their salts, characterized in that a product of formula (II):

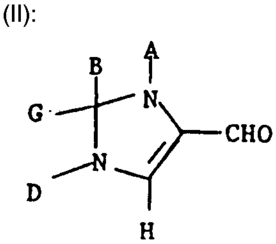 (II)

in which A, B, G and D are defined as previously, is subjected to a generator of carbanions $R_1^-$ such as a Grignard reagent of formula (III):

$R_1-Mg-Br$ (III)

or an alkyllithium compound of formula ($III_A$):

$R_1 - Li$ $(III_A)$

in which formulae $R_1$ is defined as previously, in order to obtain a product of formula (IV):

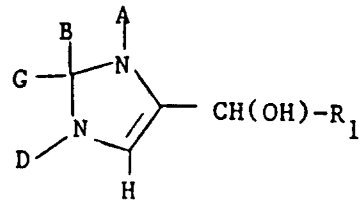

(IV)

in which A, B, G, D and $R_1$ are defined as previously, which is subjected to an oxidaxion in order to obtain the product of general formula (I) in which E represents a hydrogen atom which, if appropriate, is salified.

**7.** Preparation process for the products as defined by formula (I) of claim 1, corresponding to formula $(I_B)$:

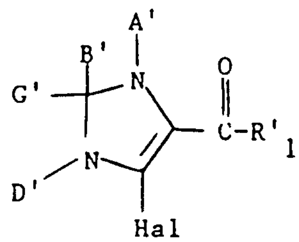

$(I_B)$

in which $R'_1$ represents a

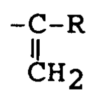

radical in which R is defined as previously or a $-C{\equiv}C-R'$ radical in which R' is defined as previously and

- either A' represents an alkyl radical containing 1 to 5 carbon atoms and B' and G' together with the carbon atom to which they are linked represent a carbonyl or thiocarbonyl radical and D' represents an alkyl radical containing 1 to 5 carbon atoms or a phenyl radical, non-substituted or substituted by one or more substituents chosen from halogen atoms, the following radicals: hydroxy, carboxy, trifluoromethyl, alkyl containing 1 to 5 carbon atoms and alkoxy containing 1 to 5 carbon atoms,
- or A' and B' together represent a bond and G' represents an alkyl or alkylthio radical containing 1 to 5 carbon atoms, and D' represents an alkyl radical containing 1 to 5 carbon atoms or a phenyl radical, non-substituted or substituted by one or more substituents chosen from halogen atoms, the following radicals: hydroxy, carboxy, trifluoromethyl, alkyl containing 1 to 5 carbon atoms and alkoxy containing 1 to 5 carbon atoms,
- or A' and B' together represent a bond and G' and D' together represent a group of formula:

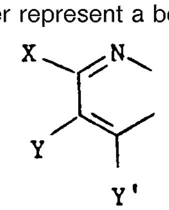

or

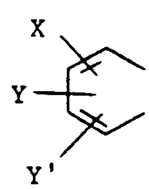

in which X, Y and Y' are defined as previously and Hal represents a halogen atom, as well as their salts, characterized in that a product of formula $(I_A)$:

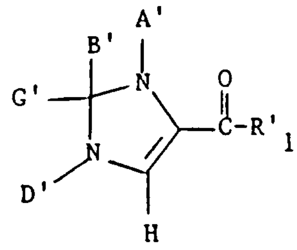

($I_A$)

in which A', B', G', D' and $R'_1$ are defined as above, is treated with an N-halosuccinimide in order to obtain a product of formula ($I_B$) which, if appropriate, is salified.

**8.** Preparation process for the products as defined by formula (I) of claim 1, corresponding to formula ($I_D$):

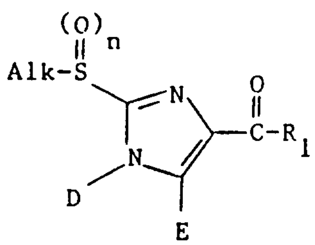

($I_D$)

in which $R_1$, D and E are defined as previously, Alk represents an alkyl radical containing 1 to 5 carbon atoms and n has the value 1 or 2 as well as their salts, characterized in that a product of formula ($I_C$):

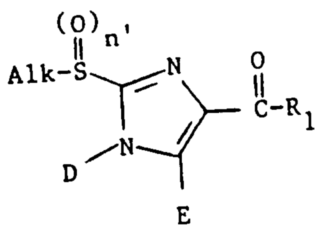

($I_C$)

in which $R_1$, D, E and Alk are defined as above and n' has the value 0 or 1, is oxidized in order to obtain a product of formula ($I_D$) which, if appropriate, is salified.

**9.** Preparation process for the products as defined by formula (I) of claim 1 in which at least one of the substituents $R_1$ and D contain a hydroxy radical, characterized in that the corresponding product of formula (V):

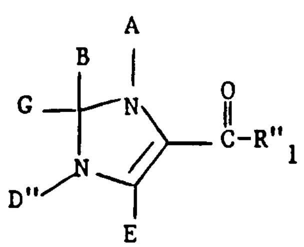

(V)

is deprotected, in which A, B, G and E are defined as previously, D'' either has the meaning indicated for D, or when it is desired to obtain a product of formula (I) in which D represents a free hydroxy radical, D'' represents a precursor of D containing a protected hydroxy radical and $R''_1$ either has the meaning indicated for $R_1$, or when it is desired to obtain a product of formula (I) in which $R_1$ represents

a free hydroxy radical, $R''_1$ represents a precursor of $R_1$ containing a protected hydroxy radical, it being understood that at least one of D'' and R'' represents a precursor of D and of $R_1$ respectively containing a protected hydroxy radical in order to obtain a product of formula (I) in which $R_1$ and D are defined as above which, if appropriate, is salified.

**10.** Preparation process according to any one of claims 6 to 9, characterized in that:
- the oxidation of the products of formula (IV) is preferably carried out with manganese dioxide;
- the oxidation of the products of formula ($I_C$) is preferably carried out with sodium metaperiodate.

**11.** Medicaments, characterized in that they are constituted by new imidazole derivatives as defined in claim 1, as well as by their addition salts with pharmaceutically acceptable acids.

**12.** Medicaments, characterized in that they are constituted by new imidazole derivatives as defined in any one of claims 2, 3 or 4, as well as by their addition salts with pharmaceutically acceptable acids.

**13.** Medicaments, characterized in that they are constituted by new imidazole derivatives as defined in claim 5, as well as by their addition salts with pharmaceutically acceptable acids.

**14.** Pharmaceutical compositions, characterized in that they contain as active ingredient, at least one of the medicaments as defined in any one of claims 11, 12 and 13.

**Patentansprüche**

**1.** Neue Imidazol-Derivate und deren Additionssalze mit Säuren, dadurch gekennzeichnet, daß sie der Formel (I)

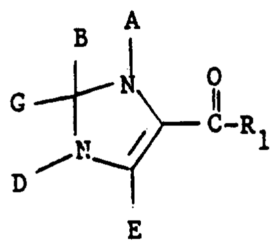

(I)

entsprechen, worin $R_1$ entweder einen Rest

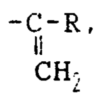

worin R für ein Wasserstoffatom oder für einen Alkylrest mit 1 bis 5 Kohlenstoffatomen steht oder einen Rest $-C \equiv C-R'$ wiedergibt, worin R' ein Wasserstoffatom, einen Alkylrest mit 1 bis 5 Kohlenstoffatomen, einen Hydroxyalkylrest mit 1 bis 5 Kohlenstoffatomen oder einen Arylrest mit 6 bis 10 Kohlenstoffatomen, unsubstituiert oder substituiert durch einen oder mehrere Substituenten, ausgewählt unter den Halogenatomen, den Hydroxy-, Carboxy-, Trifluormethyl-, Alkyl- mit 1 bis 5 Kohlenstoffatomen und Alkoxyresten mit 1 bis 5 Kohlenstoffatomen, bedeutet, und
- entweder A für einen Alkylrest mit 1 bis 5 Kohlenstoffatomen steht, und B und G gemeinsam mit dem Kohlenstoffatom, an das sie gebunden sind, einen Carbonyl- oder Thiocarbonylrest bilden, und D einen Alkylrest mit 1 bis 5 Kohlenstoffatomen oder einen Phenylrest, unsubstituiert oder substituiert durch einen oder mehrere Substituenten, ausgewählt unter den Halogenatomen, den Hydroxy-, Carboxy-, Trifluormethyl-, Alkyl- mit 1 bis 5 Kohlenstoffatomen und Alkoxyresten mit 1 bis 5 Kohlenstoffatomen, bedeutet,
- oder A und B gemeinsam eine Bindung wiedergeben, und G einen Alkyl-, Alkoxy-, Alkylthio-, Alkylsulfinyl- oder Alkylsulfonylrest mit 1 bis 5 Kohlenstoffatomen bedeutet, D für eine Alkylgruppe mit 1 bis 5 Kohlenstoffatomen oder einen Phenylrest, unsubstituiert oder substituiert durch einen oder mehrere Substituenten, ausgewählt unter den Halogenatomen, den Hydroxy-,

Carboxy–, Trifluormethyl–, Alkyl– mit 1 bis 5 Kohlenstoffatomen und Alkoxyresten mit 1 bis 5 Kohlenstoffatomen, steht,

– oder A und B gemeinsam eine Bindung bilden, und G und D gemeinsam eine Gruppe der Formel

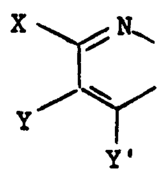

oder

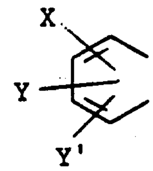

darstellen, worin X eine Alkoxy– oder Alkylthiogruppe bedeutet, und Y und Y', identisch oder voneinander verschieden, ein Wasserstoffatom oder einen Alkylrest mit 1 bis 5 Kohlenstoffatomen wiedergeben, E ein Wasserstoff– oder Halogenatom bedeutet, mit der Maßgabe, daß, wenn A und B gemeinsam eine Bindung darstellen und G und D gemeinsam eine Gruppe

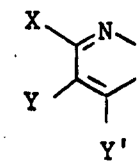

wiedergeben, E kein Wasserstoffatom bedeuten kann, wenn $R_1$ einen Rest

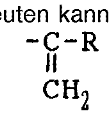

wiedergibt.

2. Derivate der Formel (I) gemäß Anspruch 1 sowie deren Salze, dadurch gekennzeichnet, daß in der Formel (I) $R_1$ einen Rest

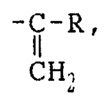

worin R wie in Anspruch 1 definiert ist, oder einen Rest –C≡C–R', worin R' wie in Anspruch 1 definiert ist, bedeutet, und E für ein Halogenatom steht.

3. Derivate der Formel (I) gemäß Anspruch 1 oder 2 sowie deren Salze, dadurch gekennzeichnet, daß in der Formel (I) A und B gemeinsam eine Bindung wiedergeben, und G und D gemeinsam eine Gruppe der Formel

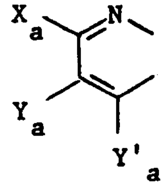

oder

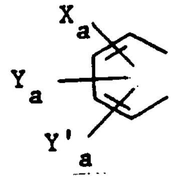

darstellen, worin $X_a$ für einen Methoxy– oder Methylthiorest steht, und $Y_a$ und $Y'_a$, identisch oder voneinander verschieden, ein Wasserstoffatom oder einen Methyl– oder Ethylrest bedeuten, und E ein Halogenatom darstellt.

4. Derivate der Formel (I) gemäß Anspruch 1 oder 2 sowie deren Salze, dadurch gekennzeichnet, daß in der Formel (I) A und B gemeinsam eine Bindung wiedergeben, G für eine Alkylthiogruppe steht, und D einen Phenylrest, unsubstituiert oder substituiert durch einen oder mehrere Substituenten, ausgewählt unter den Halogenatomen, den Hydroxy–, Carboxy–, Trifluormethyl–, Alkyl– mit 1 bis 5 Kohlenstoffatomen und Alkoxyresten mit 1 bis 5 Kohlenstoffatomen, bedeutet.

5. Derivate der Formel (I) gemäß Anspruch 1 mit den folgenden Bezeichnungen:
   - 1–(3–Brom–6–ethyl–5–methyl–7–methylthioimidazo–[1,2a]–pyrimidin–2–yl)–2–methyl–2–propen–1–on;
   - 1–[5–Brom–1–(4–ethylphenyl)–2–methylthioimidazol–4–yl]–2–methyl–2–propen–1–on;
   - 1–[5–Brom–1–(4–methoxyphenyl)–2–methylthioimidazol–4–yl]–2–propen–1–on;
   - 1–(3–Brom–6–ethyl–7–methoxy–5–methylimidazo–[1,2a]–pyrimidin–2–yl)–2–propen–1–on; und
   - 1–(3–Brom–6–ethyl–7–methoxy–5–methylimidazo–[1,2a]–pyrimidin–2–yl)–3–phenyl–2–propin–1–on;

   und deren Additionssalze mit Säuren.

6. Verfahren zur Herstellung der Produkte, definiert durch die Formel (I) gemäß Anspruch 1, entsprechend der Formel

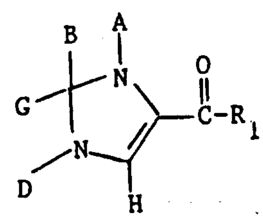

worin A, B, G, D und $R_1$ wie vorstehend definiert sind, sowie von deren Salzen, dadurch gekennzeichnet, daß man ein Produkt der Formel (II)

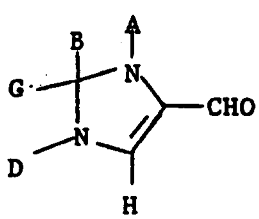

(II)

worin A, B, G und D wie vorstehend definiert sind, einem Erzeuger von Carbanionen $R_1^-$ wie einem Grignard–Reagens der Formel (III)

$R_1-Mg-Br$ (III)

oder einer Alkyllithiumverbindung der Formel ($III_A$)

$R_1-Li$ ($III_A$)

worin $R_1$ wie vorstehend definiert ist, unterzieht, um zu einem Produkt der Formel (IV)

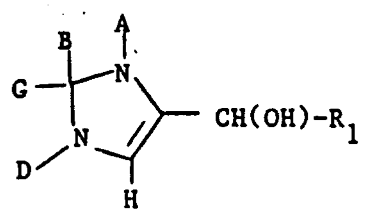

(IV)

worin A, B, G, D und $R_1$ wie vorstehend definiert sind, zu gelangen, welches man einer Oxidation unterwirft, um das Produkt der allgemeinen Formel (I), worin E ein Wasserstoffatom bedeutet, zu erhalten, welches man gegebenenfalls in ein Salz überführt.

**7.** Verfahren zur Herstellung der Produkte, wie durch Formel (I) gemäß Anspruch 1 definiert, entsprechend der Formel ($I_B$)

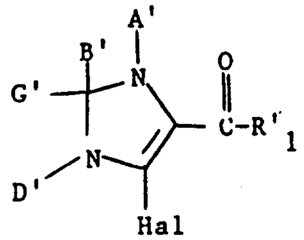

($I_B$)

worin $R'_1$ einen Rest

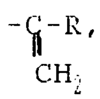

worin R wie vorstehend definiert ist, oder einen Rest $-C{\equiv}C-R'$, worin R' wie vorstehend definiert ist, bedeutet, und

- entweder A' einen Alkylrest mit 1 bis 5 Kohlenstoffatomen darstellt, und B' und G' gemeinsam mit dem Kohlenstoffatom, an das sie gebunden sind, einen Carbonyl− oder Thiocarbonylrest wiedergeben, und D' eine Alkylgruppe mit 1 bis 5 Kohlenstoffatomen oder einen Phenylrest, unsubstituiert oder substituiert durch einen oder mehrere Substituenten, ausgewählt unter den Halogenatomen, den Hydroxy−, Carboxy−, Trifluormethyl−, Alkyl− mit 1 bis 5 Kohlenstoffatomen und Alkoxyresten mit 1 bis 5 Kohlenstoffatomen, bedeutet,
- oder A' und B' gemeinsam eine Bindung bilden, und G' für einen Alkyl− oder Alkylthiorest mit 1 bis 5 Kohlenstoffatomen steht, und D' einen Alkylrest mit 1 bis 5 Kohlenstoffatomen oder einen Phenylrest, unsubstituiert oder substituiert durch einen oder mehrere Substituenten, ausgewählt unter den Halogenatomen, den Hydroxy−, Carboxy−, Trifluormethyl−, Alkyl− mit 1 bis 5 Kohlenstoffatomen und Alkoxyresten mit 1 bis 5 Kohlenstoffatomen, bedeutet,
- oder A' und B' gemeinsam eine Bindung bilden, und G' und D' gemeinsam eine Gruppe der Formel

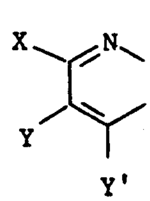

oder

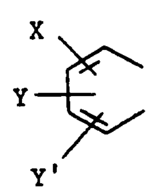

bedeutet, worin X, Y und Y' wie vorstehend definiert sind, und Hal für ein Halogenatom steht, sowie von deren Salzen, dadurch gekennzeichnet, daß man ein Produkt der Formel ($I_A$)

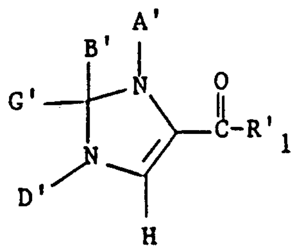

$(I_A)$

worin A', B', G', D' und $R'_1$ wie vorstehend definiert sind, mit einem N-Halosuccinimid behandelt, um zu einem Produkt der Formel $(I_B)$ zu gelangen, welches man gegebenenfalls in ein Salz überführt.

**8.** Verfahren zur Herstellung der Produkte, wie durch die Formel (I) gemäß Anspruch 1 definiert, entsprechend der Formel $(I_D)$

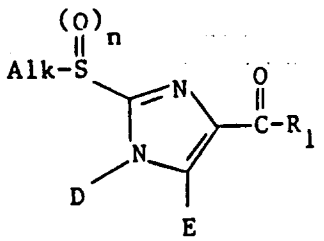

$(I_D)$

worin $R_1$, D und E wie vorstehend definiert sind, Alk für einen Alkylrest mit 1 bis 5 Kohlenstoffatomen steht, und n die Bedeutung 1 oder 2 besitzt, sowie von deren Salzen, dadurch gekennzeichnet, daß man ein Produkt der Formel $(I_C)$

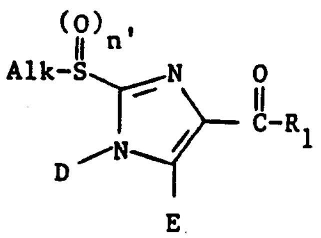

$(I_C)$

worin $R_1$, D, E und Alk wie vorstehend definiert sind, und n' die Bedeutung 0 oder 1 besitzt, oxidiert, um zu einem Produkt der Formel $(I_D)$ zu gelangen, welches man gegebenenfalls in ein Salz überführt.

**9.** Verfahren zur Herstellung der Produkte, wie durch die Formel (I) gemäß Anspruch 1 definiert, worin zumindest einer der Substituenten $R_1$ und D eine Hydroxygruppe umfaßt, dadurch gekennzeichnet, daß man an dem entsprechenden Produkt der Formel (V)

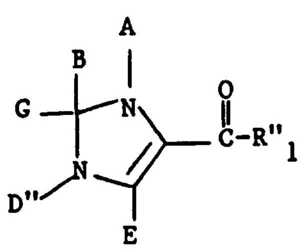

(V)

worin A, B, G und E wie vorstehend definiert sind, D" die vorstehend für D angegebene Bedeutung besitzt, oder wenn man ein Produkt der Formel (I) erhalten möchte, worin D eine freie Hydroxygruppe

bedeutet, D'' einen Vorläufer von D darstellt, der eine geschützte Hydroxygruppe enthält, und $R''_1$ entweder die für $R_1$ angegebene Bedeutung besitzt, oder wenn man ein Produkt der Formel (I) erhalten möchte, worin $R_1$ eine freie Hydroxygruppe darstellt, $R''_1$ einen Vorläufer von $R_1$ darstellt, der eine geschützte Hydroxygruppe besitzt, mit der Maßgabe, daß zumindest eines von D'' und R'' einen Vorläufer von D bzw. $R_1$ darstellt, welcher eine geschützte Hydroxygruppe enthält, eine Schutzgruppenentfernung vornimmt, um zu einem Produkt der Formel (I) zu gelangen, worin $R_1$ und D wie vorstehend definiert sind, das man gegebenenfalls einer Salzbildung unterzieht.

**10.** Verfahren gemäß einem der Ansprüche 6 bis 9, dadurch gekennzeichnet, daß
- die Oxidation der Produkte der Formel (IV) in Anwesenheit von Mangandioxid durchgeführt wird;
- die Oxidation der Produkte der Formel ($I_C$) vorzugsweise mit Natriummetaperiodat durchgeführt wird.

**11.** Arzneimittel, dadurch gekennzeichnet, daß sie aus den neuen Imidazol-Derivaten, wie in Anspruch 1 definiert, sowie aus deren Additionssalzen mit pharmazeutisch verträglichen Säuren bestehen.

**12.** Arzneimittel, dadurch gekennzeichnet, daß sie aus den neuen Imidazol-Derivaten, wie in einem der Ansprüche 2, 3 oder 4 definiert, sowie aus deren Additionssalzen mit pharmazeutisch verträglichen Säuren bestehen.

**13.** Arzneimittel, dadurch gekennzeichnet, daß sie aus den neuen Imidazol-Derivaten, wie in Anspruch 5 definiert, sowie aus deren Additionssalzen mit pharmazeutisch verträglichen Säuren bestehen.

**14.** Pharmazeutische Zusammensetzungen, dadurch gekennzeichnet, daß sie als Wirkstoff zumindest eines der Arzneimittel, wie in einem der Ansprüche 11, 12 und 13 definiert, enthalten.